(19) Europäisches Patentamt
European Patent Office
Office européen des brevets

(11) Veröffentlichungsnummer: **0 193 828**
**B1**

(12) # EUROPÄISCHE PATENTSCHRIFT

(45) Veröffentlichungstag der Patentschrift: **27.04.88**

(51) Int. Cl.[4]: **C 08 G 18/79**, C 07 D 251/34, C 09 D 3/72

(21) Anmeldenummer: **86102373.7**

(22) Anmeldetag: **24.02.86**

(54) **Isocyanuratgruppen aufweisende Polyisocyanate, ein Verfahren zu ihrer Herstellung und ihre Verwendung als Isocyanatkomponente in Polyurethanlacken.**

(30) Priorität: **05.03.85 DE 3507719**

(43) Veröffentlichungstag der Anmeldung: **10.09.86 Patentblatt 86/37**

(45) Bekanntmachung des Hinweises auf die Patenterteilung: **27.04.88 Patentblatt 88/17**

(84) Benannte Vertragsstaaten: **AT BE CH DE FR GB IT LI NL SE**

(56) Entgegenhaltungen: **EP-A-0 047 452**

(73) Patentinhaber: **BAYER AG, Konzernverwaltung RP Patentabteilung, D-5090 Leverkusen 1 Bayerwerk (DE)**

(72) Erfinder: **Halpaap, Reinhard, Dr., Kleinfeldchensweg 37b, D-5000 Köln 91 (DE)**
Erfinder: **Klein, Gerhard, Dr., v.- Flotow- Strasse 7, D-4019 Monheim (DE)**
Erfinder: **Richter, Roland, Dr., Roggendorfstrasse 53, D-5000 Köln 80 (DE)**
Erfinder: **Müller, Hanns Peter, Dr., Im Kerberich 6, D-5068 Odenthal (DE)**
Erfinder: **Pedain, Josef, Dr., Haferkamp 6, D-5000 Köln 80 (DE)**
Erfinder: **Kreuder, Hans- Joachim, Dr., Doerperhofstrasse 35, D-4150 Krefeld (DE)**

EP 0 193 828 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Isocyanuratgruppen aufweisende Polyisocyanate auf Basis ausgewählter Diisocyanate, ein Verfahren zu ihrer Herstellung durch Trimisierung eines Teils der Isocyanatgruppen von speziellen aliphatisch-cycloaliphatischen Diisocyanaten und die Verwendung der Isocyanato-isocyanurate als Isocyanatkomponente in Polyurethanlacken.

Isocyanuratgruppen aufweisende (cyclo-)aliphatische Polyisocyanate sind als Lackpolyisocyanate technisch von größtem Interesse. Während niedermolekulare aliphatische Diisocyanate wegen ihrer noch relativ hohen Flüchtigkeit aufgrund ihrer toxikologischen Eigenschaften als solche in Lackbindemitteln nicht verwendet werden können, weisen Isocyanato-isocyanurate eine Reihe von Vorteilen auf. Es handelt sich um Oligomere mit einem niedrigen Dampfdruck, die weitgehend monomerenfrei sind. Sie haben eine Isocyanatfunktionalität $\geqslant 3$, so daß sich ein hoher Vernetzungsgrad erreichen läßt. Als aliphatische Polyisocyanate bewirken sie eine gute Lichtechtheit der aus ihnen erhaltenen Beschichtungen.

Bezüglich der Herstellung von Isocyanuratgruppen aufweisenden Polyisocyanaten sind die unterschiedlichsten Verfahren bekannt geworden. Diese bekannten Verfahren unterscheiden sich in der Regel durch die Auswahl der speziellen Trimerisierungskatalysatoren oder auch durch die Auswahl der bei der Trimerisierungsreaktion einzusetzenden Polyisocyanate (vgl. z. B. GB-PS-1 391 066, GB-PS-1 386 399, DE-OS-2 325 826, DE-OS-2 616 415, DE-OS-2 806 731, DE-OS-2 901 479, DE-OS-3 100 262, DE-OS-3 219 608, EP-OS-17 998, EP-OS-33 581, EP-OS-57 653, EP-OS-89 297, EP-OS-82 987 oder EP-OS-100 129).

Alle diese Verfahren haben einen wesentlichen prinzipiellen Nachteil. Um niedrigviskose oder in Lacklösungsmitteln gut lösliche Isocyanato-isocyanurate zu erhalten, muß die Trimerisierungsreaktion bereits bei einem relativ niedrigen Trimerisierungsgrad (Trimerisierungsgrad = Prozentsatz der in den Ausgangsisocyanaten vorliegenden Isocyanatgruppen, die unter Trimerisierung abreagiert werden) abgebrochen werden und zwecks Erzielung eines monomerenarmen Trimerisierungsprodukts eine in der Regel apparativ aufwendige Dünnschichtdestillation durchgeführt werden. Andernfalls würden neben den in erster Linie angestrebten, niedrigviskosen bzw. gut löslichen monomeren Isocyanuraten größere Mengen an Oligoisocyanuraten (Polyisocyanate mit Isocyanuratstruktur, die pro Molekül mehr als einen Isocyanuratring aufweisen) entstehen. Dies ist darauf zurückzuführen, daß die Isocyanatgruppen der Ausgangsdiisocyanate in der Regel eine gleichartige oder eine nur geringfügig unterschiedliche Reaktivität aufweisen, so daß nicht ausgeschlossen werden kann, daß jeweils nur eine Isocyanatgruppe der Ausgangsdiisocyanate unter Isocyanuratbildung abreagiert.

Es war daher die der Erfindung zugrundeliegende Aufgabe, neue als Lackpolyisocyanate geeignete Isocyanuratgruppen aufweisende Polyisocyanate zur Verfügung zu stellen, die im wesentlichen keine oligomeren Trimerisierungsprodukte der genannten Art aufweisen, d.h. die im wesentlichen aus Tris-isocyanato-monoisocyanuraten bestehen, und bei deren Herstellung die Trimerisierungsreaktion nicht vorzeitig, d.h. bei einem Trimerisierungsgrad von ca. 20 - 40 % abgebrochen werden muß, um dieses Ziel zu erreichen.

Diese Aufgabe konnte durch die Bereitstellung der nachstehend näher beschriebenen erfindungsgemäßen Isocyanato-isocyanurate bzw. des Verfahrens zur ihrer Herstellung, bei welchem ganz bestimmte Ausgangsdiisocyanate eingesetzt werden, gelöst werden.

Gegenstand der Erfindung sind Isocyanato-isocyanurate der allgemeinen Formel

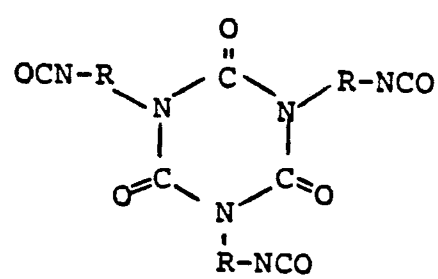

in welcher

R für einen Rest steht, wie er durch Entfernung der Isocyanatgruppen von einem aliphatisch-cycloaliphatischen Diisocyanat erhalten wird, welches eine sterisch ungehinderte, an ein primäres aliphatisches Kohlenstoffatom gebundene Isocyanatgruppe und eine sterisch gehinderte Isocyanatgruppe, die an ein tertiäres Kohlenstoffatom, das Teil eines cycloaliphatischen Ringsystems ist, aufweist, wobei die Isocyanatgruppen in der besagten Formel mit dem besagten tertiären Kohlenstoffatom verknüpft sind.

Gegenstand der Erfindung ist auch ein Verfahren zur Herstellung von Isocyanuratgruppen aufweisenden Polyisocyanaten durch katalytische Trimerisierung eines Teil der Isocyanatgruppen von organischen Diisocyanaten und gegebenenfalls Abstoppung der Trimerisierungsreaktion beim jeweils gewünschten Trimerisierungsgrad durch Zugabe eines Katalysatorengifts und/oder durch thermische Desaktivierung des eingesetzten Katalysators, dadurch gekennzeichnet, daß man als Ausgangsdiisocyanate solche einsetzt, die bei einem NCO-Gehalt von 29 bis 50 Gew.-% eine sterisch ungehinderte, an ein primäres aliphatisches

Kohlenstoffatom gebundene Isocyanatgruppe und eine sterisch gehinderte Isocyanatgruppe, die an ein tertiäres Kohlenstoffatom, das Teil eines cycloaliphatischen Ringsystems ist, aufweisen.

Gegenstand der Erfindung ist schließlich auch die Verwendung der erfindungsgemäßen Isocyanuratgruppen aufweisenden Polyisocyanate, gegebenenfalls in mit Blockierungsmitteln für Isocyanatgruppen blockierter Form, als Isocyanatkomponente in Polyurethanlacken.

Erfindungswesentlich ist die Verwendung von speziellen Ausgangsdiisocyanaten bei der Trimerisierungsreaktion. Es handelt sich bei den Ausgangsdiisocyanaten um aliphatisch-cycloaliphatische Diisocyanate mit einem NCO-Gehalt von 20 bis 50 Gew.-%, die neben einer sterisch ungehinderten, aliphatisch gebundenen Isocyanatgruppe eine sterisch gehinderte, cycloaliphatisch gebundene Isocyanatgruppe aufweisen. Gut geeignete erfindungsgemäße Ausgangsdiisocyanate sind solche der Formel

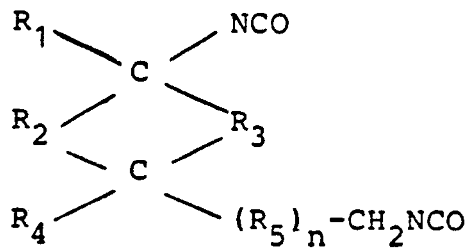

I

für welche

$R_1$ für einen Alkylrest mit 1 bis 4 Kohlenstoffatomen, vorzugsweise einen Methylrest steht,

$R_2$ und $R_3$ für gleiche oder verschiedene Reste stehen und jeweils einen zweiwertigen linearen oder verzweigten gesättigten Kohlenwasserstoffrest mit 1 bis 4, vorzugsweise 1 bis 3 Kohlenstoffatomen bedeuten, wobei die Summe der Kohlenstoffatome dieser Reste vorzugsweise 3 bis 6, insbesondere 3 oder 4 beträgt,

$R_4$ für Wasserstoff oder ein Alkylrest mit 1 bis 4 Kohlenstoffatomen, vorzugsweise für Wasserstoff oder einen Methylrest steht,

$R_5$ für einen zweiwertigen, linearen oder verzweigten, gesättigten aliphatischen Kohlenwasserstoffrest mit 1 bis 4 Kohlenstoffatomen steht und

n für 0 oder 1 steht.

Besonders bevorzugte Diisocyanate sind z. B. 1-Isocyanato-1-methyl-4(3)-isocyanatomethylcyclohexan, welches im allgemeinen als Gemisch der 4- und 3-Isocyanatomethyl-Isomeren vorliegt, 1-Isocyanato-1-methyl-4-(4-isocyanatobut-2-yl)-cyclohexan oder 1-Isocyanato-1,2,2-trimethyl-3-(2-isocyanatoethyl)-cyclopentan.

Die Diisocyanate können beispielsweise hergestellt werden, indem man ungesättigte Amine der allgemeinen Formel II

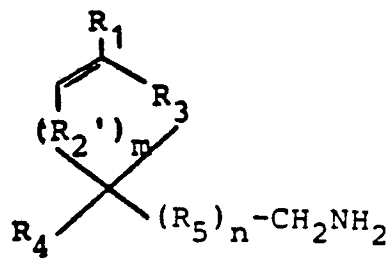

II

in welcher

$R_2'$ für einen zweiwertigen, gesättigten, linearen oder verzweigten Kohlenwasserstoffrest mit 1 bis 3 Kohlenstoffatomen steht,

m für 0 oder 1 steht und

$R_1$, $R_3$, $R_4$, $R_5$ und n die obengenannte Bedeutung haben, oder Aminoalkohole der allgemeinen Formel III,

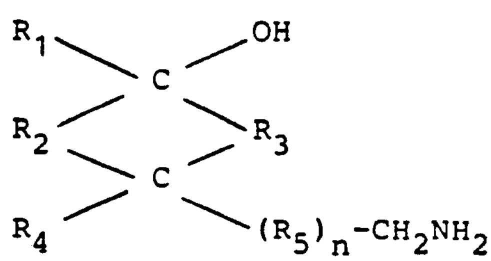

III

in welcher $R_1$, $R_2$, $R_3$, $R_4$, $R_5$ und n die oben angegebene Bedeutung haben, in einer Ritter-Reaktion mit Blausäure zu den Diaminen der allgemeinen Formel IV,

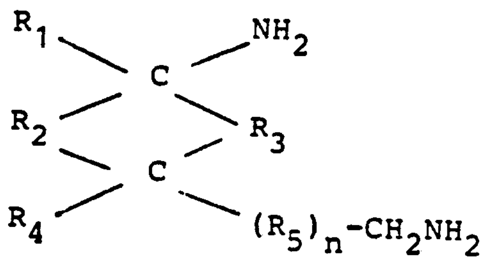

IV

umsetzt.

Aus den Diaminen der allgemeinen Formel IV erhält man durch Phosgenierung die Diisocyanate der allgemeinen Formel I.

Die ungesättigten Amine der allgemeinen Formel II sind entweder bekannt oder können aus Verbindungen der allgemeinen Formel V,

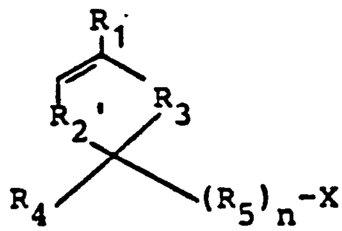

V

in welcher
X für -CHO oder -CN steht und
$R_1$, $R_2'$, $R_3$, $R_4$, $R_5$ und n die bereits obengenannte Bedeutung haben,
durch katalytische Hydrierung gewonnen werden.

Die Grundsubstanzen der allgemeinen Formel V können beispielsweise durch die bekannte Diels-Alder-Reaktion aus den entsprechenden, konjugierte Doppelbindungen aufweisenden Bisolefinen und ungesättigten Nitrilen oder Aldehyden oder durch Hydroformylierung der entsprechenden ungesättigten Kohlenwasserstoffe erhalten werden. So stellt beispielsweise das als Stellungsisomerengemisch vorliegende Diels-Alder-Adukkt der Formel VIa und VIb

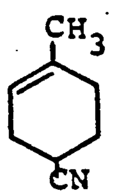

VIa

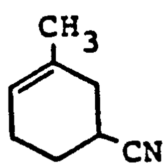

VIb

die Grundsubstanz des ebenfalls als Stellungsisomerengemisch vorliegenden 1-Isocyanato-1-methyl-4(3)-iso-cyanatomethylcyclohexans und die durch Hydroformylierung von Limonen erhältliche Verbindung der Formel VII

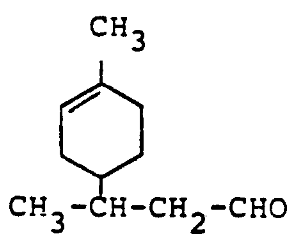

VII

die Grundsubstanz von 1-Isocyanato-1-methyl-4(4-isocyanatobut-2-yl)-cyclohexan dar. Die entsprechende Grundsubstanz von 1-Isocyanato-1,2,2-trimethyl-3-(2-isocyanatoethyl)-cyclopentan ist Campholenaldehyd der Formel VIII

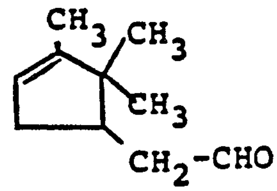

VIII

Weitere, der allgemeinen Formel V entsprechende, Grundsubstanzen können in Analogie zu diesen Ausführungen durch geeignete Auswahl der zu ihrer Herstellung benutzten Ausgangsmaterialien erhalten werden. Die Verbindungen VI-VIII sind im übrigen literaturbekannt (VI: Chem. Abstr. 71, 112475 F; VII: EP-A-000859; VIII: Berichte 68B, 1430 (1935)).

Die Ritter-Reaktion der ungesättigten Amine der allgemeinen Formel II oder der Aminoalkohole der allgemeinen Formel III wird in Gegenwart einer starken Säure wie Schwefelsäure, Phosphorsäure, Alkyl- oder Arylsulfonsäuren oder Trifluoressigsäure durchgeführt. Bevorzugt wird Schwefelsäure verwendet. Der Wassergehalt der Säure kann zwischen 5 und 50 % betragen, vorzugsweise jedoch 25 bis 35 %. Pro Mol ungesättigtes Amin verwendet man 1 bis 3 Mol Säure, vorzugsweise 2 Mol. Bezogen auf das ungesättigte Amin der allgemeinen Formel II oder den Aminoalkohol der allgemeinen Formel III wird eine äquimolare Menge oder ein Überschuß von bis zu einem Mol Blausäure verwendet. In einer bevorzugten Verfahrensweise wird das ungesättigte Amin der allgemeinen Formel II zu der Säure gegeben und anschließend die Blausäure zugefügt. Die Temperatur wird während der Zugabe des Amins zwischen 0 und 25°C und während der Blausäurezugabe zwischen 10 und 50°C, vorzugsweise zwischen 30 und 45°C gehalten. Nach einer Reaktionszeit von 2 bis 10 Stunden, vorzugsweise 4 bis 6 Stunden wird das gebildete Formamid sauer hydrolysiert und das gebildete Diamin der allgemeinen Formel IV durch Neutralisation mit einer Base wie z. B. Natronlauge freigesetzt.

Das durch die Ritter-Reaktion gewonnene Diamin der allgemeinen Formel IV kann in an sich bekannter Weise phosgeniert werden. Dazu wird z. B. das Diamin in einem inerten Lösungsmittel mit Kohlendioxid bei Temperaturen zwischen 0 und 150°C, vorzugsweise zwischen 0 und 100°C gesättigt. Das entstandene Additionsprodukt wird dann bei 0 bis 200°C, vorzugsweise bei 120 bis 150°C mit Phosgen zum Diisocyanat der allgemeinen Formel I umgesetzt. Es können alle indifferenten Lösungsmittel verwendet werden, deren Siedetemperatur für die Phosgenierung hoch genug liegt und die zum Diisocyanat eine ausreichende Siedepunktdifferenz aufweisen. Bevorzugt sind Chlorbenzole, Nitrobenzole, Xylole, Tetralin und Decalin.

In einer anderen Ausführungsform der Phosgenierung wird das Diamin in einem indifferenten Lösungsmittel zu einer Lösung von Phosgen in demselben Lösungsmittel bei Temperaturen zwischen -20°C und +50°C gegeben. Der Phosgenüberschuß bezogen auf das Diamin sollte 2 bis 10 Mol betragen, vorzugsweise 4 bis 6 Mol. Eine weitere Umsetzung des Additionsprodukts zum Diisocyanat erfolgt dann bei einer Temperatur von 20 bis 200°C, vorzugsweise zwischen 120°C und 150°C.

Die auf diese Weise erhaltenen Diisocyanate weisen einen NCO-Gehalt von 20 bis 50, vorzugsweise von 30 bis 48 Gew.-% auf und stellen im allgemeinen Gemische von Stereoisomeren dar. Darüber hinaus kann es sich bei den Diisocyanaten, insbesondere im Falle der Verwendung von ungesättigten Nitrilen der allgemeinen Formel V als Grundsubstanz, die durch Diels-Alder-Reaktion erhalten worden sind, um Gemische von Stellungsisomeren handeln.

Besonders bevorzugt werden als erfindungsgemäß einzusetzende Ausgangsdiisocyanate 4-Isocyanatomethyl-1-methyl-cyclohexanisocyanat oder 3-Isocyanatomethyl-1-methyl-cyclohexanisocyanat oder ein Gemisch der beiden Isomeren, besonders bevorzugt in einem Gewichtsverhältnis der 4- und 3-Isomeren von 70 : 30 bis 90 : 10, und/oder 4-(4-Isocyanatobut-2-yl)-1-methylcyclohexanisocyanat verwendet.

Für das erfindungsgemäße Verfahren geeignete Trimerisierungskatalysatoren sind alle beliebigen bisland für diesen Zweck eingesetzten Verbindungen wie beispielsweise Phosphine der in DE-OS-1 934 763 beschriebenen Art, Alkaliphenolate der in GB-PS-1 391 066 oder GB-PS-1 386 399 beschriebenen Art, Aziridinderivate in Kombination mit tertiären Aminen der in DE-OS-2 325 826 beschriebenen Art, Mannichbasen, beispielsweise auf Basis von i-Nonylphenol, Formaldehyd und Dimethylamin der in US-PS-4 115 373 beschriebenen Art, quaternäre Ammoniumcarboxylate der in EP-OS-17 998 beschriebenen Art, quaternäre Ammoniumphenolate mit zwitterionischer Struktur der beispielsweise in US-PS-4 335 219 beschriebenen Art, Ammoniumphosphonate und -phosphate der in DE-OS-3 227 489 beschriebenen Art oder Alkalicarboxylate der in DE-OS-3 219 608 beschriebenen Art.

Besonders gut geeignete Katalysatoren für das erfindungsgemäße Verfahren sind basische Alkalimetallsalze in Verbindung mit Phasentransfer-Katalysatoren, wie sie von R. Richter, P. Müller und K. Wagner, Die Angewandte Makromolekulare Chemie 113, 1 - 9 (1983) näher beschrieben werden, hier besonders bevorzugt Kaliumacetat komplexiert mit einem Polyethylenglykol, welches im Mittel 5 bis 8 Ethylenoxideinheiten enthält.

Besonders gut geeignet für das erfindungsgemäße Verfahren sind als Katalysatoren außerdem quaternäre Ammoniumhydroxide der allgemeinen Formel

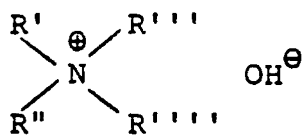 ,

wie sie in den DE-OSen 2 806 731 und 2 901 479 beschrieben sind. Bevorzugt werden dabei quaternäre Ammoniumhydroxide der angegebenen Struktur, wobei die Reste R′ bis R′′′′ für gleiche oder verschiedene Alkylgruppen mit 1 bis 20, vorzugsweise 1 bis 4, Kohlenstoffatomen stehen, die gegebenenfalls mit Hydroxylgruppen substituiert sind, und wobei zwei der genannten Reste R′ bis R′′′′ auch zusammen mit dem Stickstoffatom und gegebenenfalls mit einem weiteren Stickstoff- oder Sauerstoffatom einen heterocyclischen Ring mit 3 bis 5 Kohlenstoffatomen bilden können, oder wobei die Reste R′ bis R′′′′ jeweils für Ethylenreste stehen, die zusammen mit dem quaternären Stickstoffatom und einem weiteren tertiären Stickstoffatom ein bicyclisches Triethylendiamin-Gerüst bilden, mit der Maßgabe, daß mindestens einer der genannten Reste mindestens eine aliphatisch gebundene Hydroxylgruppe aufweist, die vorzugsweise in 2-Stellung zum quaternären Stickstoffatom angeordnet ist, wobei der hydroxylsubstituierte Rest oder die hydroxylsubstituierten Reste außer dem Hydroxyl-Substituenten auch beliebige andere Substituenten, insbesondere $C_1$ bis $C_4$-Alkoxy-Substituenten, aufweisen können. Die Darstellung der zuletzt beschriebenen Katalysatoren erfolgt in an sich bekannter Weise durch Umsetzung von tertiärem Amin mit Alkylenoxid in wäßrig-alkoholischem Medium (vgl. US-PS-3 995 997, Kol. 2, Zeilen 19 - 44). Als tertiäre Amine sind dafür beispielsweise genannt: Trimethylamin, Tributylamin, 2-Dimethylaminoethanol, Triethanolamin, Dodecyldimethylamin, N,N-Dimethylcyclohexylamin, N-Methylpyrrolidin, N-Methylmorpholin oder 1,4-Diazabicyclo-[2,2,2]-octan; als Alkylenoxide können beispielsweise Ethylenoxid, Propylenoxid, 1,2-Butylenoxid, Styroloxid oder Methoxy-, Ethoxy- oder Phenoxypropylenoxid verwendet werden. Ganz besonders bevorzugte Katalysatoren aus dieser Gruppe sind N,N,N-Trimethyl-N-(2-hydroxyethyl)-ammoniumhydroxid und N,N,N-Trimethyl-N-(2-hydroxypropyl)-ammoniumhydroxid.

Die Trimerisierungsreaktion kann zweckmäßigerweise in Abwesenheit, ohne weiteres jedoch auch in Gegenwart von gegenüber Isocyanatgruppen inerten Lösungsmitteln durchgeführt werden. Je nach Anwendungsbereich der erfindungsgemäßen Produkte können niedrig- bis mittelsiedende Lösungsmittel oder hochsiedende Lösungsmittel angewandt werden, wobei die Produkte bei Verwendung von Estern wie Ethylacetat oder Butylacetat, von Ketonen wie Aceton oder Butanon, Aromaten wie Toluol oder Xylol und Halogenkohlenwasserstoffen wie Methylenchlorid und Trichlorethylen im allgemeinen zunächst gelöst bleiben, während sie in Ethern wie Diisopropylether oder Alkanen wie Cyclohexan, Petrolether oder Ligroin in der Regel eine zweite Phase bilden oder ausfallen.

Die Trimerisierungskatalysatoren werden im allgemeinen in Mengen von 0,005 - 5 Gew.-%, vorzugsweise 0,01 - 2 Gew.-%, bezogen auf eingesetztes Diisocyanat eingesetzt. Werden zum Beispiel die bevorzugten Katalysatoren wie komplexiertes Kaliumacetat oder N,N,N-Trimethyl-N-(2-hydroxypropyl)-ammoniumhydroxid verwendet, so sind Mengen von 0,05 bis 1 Gew.-%, bevorzugt von 0,07 - 0,7 Gew.-%, bezogen auf Ausgangsdiisocyanat, im allgemeinen ausreichend. Die Katalysatoren können in reiner Form oder als Lösung eingesetzt werden. Als Lösungsmittel eignen sich je nach Art des Katalysators zum Beispiel die bereits genannten gegenüber Isocyanatgruppen inerten Lösungsmittel, ebenso auch Dimethylformamid oder Dimethylsulfoxid. Bei der Mitverwendung von Carbamidsäurederivate-bildenden Hydroxyverbindungen als Cokatalysatoren ist es von Vorteil diese gleichzeitig als Katalysatorlösungsmittel zu verwenden. Dafür eignen sich zum Beispiel Methanol, Ethanol, Isopropanol, 2-Ethylhexanol oder Glykole wie Ethandiol, 1,2-Propandiol, 1,3- und 1,4-Butandiol, 1,6- und 2,5-Hexandiol, 2-Ethyl-1,3-hexandiol oder 2,2,4-Trimethyl-1,3-pentandiol.

Die Mitverwendung von Cokatalysatoren ist beim erfindungsgemäßen Verfahren möglich, aber nicht erforderlich. Als Cokatalysatoren eignen sich im Prinzip alle Substanzen, von denen eine polymerisierende Wirkung auf Isocyanate bekannt ist und wie sie beispielsweise in der DE-OS-2 806 731 beschrieben sind. Die Cokatalysatoren werden gegebenenfalls gegenüber den verwendeten Trimerisierungskatalysatoren unterschüssig eingesetzt; eine Ausnahme bilden die beschriebenen Hydroxyverbindungen, die gleichzeitig als Carbamidsäurederivate bildende Cokatalysatoren und als Katalysatorlösungsmittel fungieren und im Überschuß gegenüber dem Katalysator verwendet werden können.

Die zur Trimerisierung erforderliche Reaktionstemperatur liegt bei dem erfindungsgemäßen Verfahren bei 20 - 200°C, bevorzugt bei 40 - 160°C, besonders bevorzugt bei 40-120°C. In Abwesenheit von inerten Lösungsmitteln wird besonders bevorzugt bei 80 - 120°C gearbeitet.

Die Durchführung des erfindungsgemäßen Verfahrens kann zum Beispiel in der nachfolgend beschriebenen Weise erfolgen:

Das Ausgangsdiisocyanat wird unter Feuchtigkeitsausschluß und gegebenenfalls unter Inertgas in einem geeigneten Rührgefäß vorgelegt und zum Beispiel mit gleichen Teilen eines gegenüber Isocyanatgruppen inerten Lösungsmittels wie z. B. Toluol, Butylacetat, Diisopropylether oder Cyclohexan vermischt. Nun wird bei zum Beispiel 60°C mit dem erforderlichen Katalysator oder der Katalysatorlösung versetzt, wobei augenblicklich die Trimerisierung beginnt und durch die exotherme Reaktion angezeigt wird. Man hält das 4(3)-Isocyanatomethyl-1-methylcyclohexanisocyanat oder das 4-(4-Isocyanatobut-2-yl)-1-methylcyclohexanisocyanat bei beispielsweise 80°C und verfolgt den Fortlauf der Reaktion durch NCO-Gehaltstitration. Die Reaktion wird dann beim jeweils erwünschten Trimerisierungsgrad abgebrochen.

Vorzugsweise erfolgt diese Beendigung der Trimerisierungsreaktion bei einem Trimerisierungsgrad von 50 %, d.h. wenn der Gehalt des Reaktionsansatzes an Isocyanatgruppen auf 50 % des Ausgangswerts abgefallen ist. Zu diesem Zeitpunkt liegt der Gehalt des Reaktionsansatzes an freiem monomerem Diisocyanat im allgemeinen unter 2, vorzugsweise unter 1 Gew.-%, während, wie gelchromatographische Untersuchungen zeigten, das Trimerisierungsprodukt praktisch ausschließlich aus den erwünschten Tris-isocyanato-monoisocyanuraten besteht.

Der Abbruch der Trimerisierungsreaktion kann beispielsweise durch Zugabe eines Katalysatorengifts der in den obengenannten Literaturstellen beispielhaft genannten Art, beispielsweise bei Verwendung von basischen Katalysatoren durch Zugabe einer der Katalysatorenmenge mindestens äquivalenten Menge eines Säurechlorids wie Benzoylchlorid erfolgen. Bei Verwendung von Hitze-labilen Katalysatoren, beispielsweise von quaternären Ammoniumhydroxiden der obengenannten Art kann auch auf eine Vernichtung des Katalysators durch Zugabe eines Katalysatorengifts verzichtet werden, da sich diese Katalysatoren im Lauf der Reaktion selbst zersetzen. Im Falle der Verwendung derartiger Katalysatoren wird die Katalysatorenmenge und die Reaktionstemperatur vorzugsweise so gewählt, daß der sich kontinuierlich zersetzende Katalysator bei Erreichen des genannten Trimerisierungsgrads verbraucht, d.h. zersetzt ist. Die hierzu erforderliche Menge an Katalysator bzw. die hierzu erforderliche Reaktionstemperatur kann in einem orientierenden Vorversuch ermittelt werden. Es ist auch möglich, zunächst eine geringere Menge eines Hitze-sensiblen Katalysators einzusetzen als zu Erreichen des angestrebten Trimerisierungsgrades erforderlich ist und die Umsetzung im Verlauf der Trimerisierungsreaktion durch weitere portionsweise Zugabe von Katalysator nachzukatalysieren, wobei die nachträglich zugesetzte Menge an Katalysator so bemessen wird, daß bei Erreichen des angestrebten Trimerisierungsgrades die Gesamtmenge an Katalysator verbraucht ist. Bei Verwendung von unpolaren Lösungsmitteln wie beispielsweise Toluol ist auch die Verwendung von suspendierten Katalysatoren möglich, die bei Erreichen des angestrebten Trimerisierungsgrades durch Abfiltrieren aus dem Reaktionsgemisch entfernt werden. Grundsätzlich kann jedoch gesagt werden, daß aufgrund der selektiven Reaktivität der Isocyanatgruppen der Ausgangsdiisocyanate die Frage der Vernichtung bzw. Entfernung des Katalysators beim angestrebten Trimerisierungsgrad weit weniger kritisch ist als bei den bekannten Verfahren des Standes der Technik, wo weit mehr als beim erfindungsgemäßen Verfahren die Möglichkeit gegeben ist, daß auch die zweite Isocyanatgruppe des eingesetzten Ausgangsdiisocyanats unter Trimerisierung abreagiert.

Die Aufarbeitung des Reaktionsgemisches kann, event. nach vorheriger Abtrennung unlöslicher Katalysatorbestandteile, je nach vorheriger Reaktionsführung oder je nach Einsatzgebiet der verwendeten Isocyanate in verschiedener Art erfolgen. So ist es zum Beispiel vorteilhaft möglich ein in Lösung hergestelltes Isocyanatoisocyanurat ohne jeden Reinigungsschritt, insbesondere ohne Dünnschichtdestillation, bei einem Monomerengehalt $< 1$ Gew.-% direkt als Lackrohstoff weiterzuverwenden. Ebenso ist es zum Beispiel möglich ein in Substanz trimerisiertes 4(3)-Isocyanatomethyl-1-methylcyclohexanisocyanat, das nach dem Abkühlen als Festharz anfällt, direkt als monomerenarme Lackisocyanatkomponente weiterzuverarbeiten. Vorteilhaft kann aber auch zum Beispiel bei der Herstellung des Isocyanurats aus 4(3)-Isocyanatomethyl-1-methylcyclohexanisocyanat ein Lösungsmittelgemisch gewählt werden, z. B. Diisopropylether/Petrolether, in welchem das Produkt als kristallines, weißes Pulver während des Abkühlvorgangs ausfällt und abfiltriert werden kann. Man kann aber auch zum Beispiel nach vollständigem Umsatz der primär gebundenen NCO-Gruppen des 4-(4-Isocyanatobut-2-yl)-1-methylcyclohexanisocyanats das bei der Trimerisierung verwendete Lösungsmittel durch Destillation entfernen.

Die erfindungsgemäßen Produkte stellen wertvolle Ausgangsmaterialien zur Herstellung von Polyurethankunststoffen nach dem Isocyanat-Polyadditionsverfahren, insbesondere zur Herstellung von Ein- oder Zweikomponenten-Polyurethanlacken dar.

Bevorzugte Reaktionspartner für die erfindungsgemäßen, gegebenenfalls in blockierter Form vorliegenden Verfahrensprodukte bei der Herstellung von Polyurethanlacken sind die in der Polyurethanlacktechnik an sich bekannten Polyhydroxypolyester und -ether, Polyhydroxypolyacrylate und gegebenenfalls niedermolekularen, mehrwertigen Alkohole. Auch Polyamine, insbesondere in blockierter Form als Polyketimine oder Oxazolidine sind denkbare Reaktionspartner für die erfindungsgemäßen Verfahrensprodukte. Die Mengenverhältnisse, in welchen die erfindungsgemäßen, gegebenenfalls blockierten Polyisocyanate und die genannten Reaktionspartner bei der Herstellung von Polyurethanlacken umgesetzt werden, werden im allgemeinen so gewählt, daß auf eine (gegebenenfalls blockierte) Isocyanatgruppe 0,8 - 3, vorzugsweise 0,9 - 1,1, Hydroxy-, Amino- und/oder Carboxylgruppen entfallen.

Zur Beschleunigung der Aushärtung können in bekannter Weise die in der Isocyanatchemie üblichen Katalysatoren verwendet werden, wie z. B. tert. Amine wie Triethylamin, Pyridin, Methylpyridin, Benzyldimethylamin, N,N-Dimethylaminocyclohexan, N-Methylpiperidin, Pentamethyldiethylentriamin, 1,4-Diazabicyclo-[2,2,2]-octan, N,N'-Dimethylpiperazin usw., Metallsalze wie Eisen(III)-chlorid, Zinkchlorid, Zink-2-ethylcaproat, Zinn(II)-2-ethylcaproat, Dibutylzinn(IV)-dilaurat, Molybdänglykolat, usw.

Die erfindungsgemäßen Isocyanato-isocyanurate können auch als wertvolle Ausgangsmaterialien für Zweikomponenten-Polyurethan-Einbrennlacke dienen. Sie können dazu in mit den an sich bekannten Blockierungsmitteln blockierter Form eingesetzt werden. Besonders vorteilhaft ist jedoch aufgrund der Reaktionsträgheit der tertiär gebundenen Isocyanatgruppen der erfindungsgemäßen Polyisocyanate ihre mögliche Anwendung in Zweikomponenten-Einbrennlacken ohne vorherige Verkappung der Isocyanatgruppen mit Blockierungsmitteln.

Damit entfällt bei solchen Systemen der Verfahrensschritt der Blockierung, und es erweist sich als ganz

besonders günstig, daß während des Einbrennvorgangs kein Blockierungsmittel als sogeannter Abspalter freigesetzt wird. Bevorzugt kann so zum Beispiel das Isocyanato-isocyanurat des 4(3)-Isocyanatomethyl-1-methylcyclohexanisocyanats, das einen Schmelzbereich oberhalb ca. 100°C aufweist, als Abspalter-freie Isocyanatkomponente für Pulverlacke verwendet werden. Wird jedoch eine Blockierung der NCO-Gruppen gewünscht, so kann nach an sich bekannter Weise verfahren werden. Das Polyisocyanat wird mit geeigneten Blockierungsmitteln ganz oder teilweise blockiert, vorzugsweise bei erhöhter Temperatur (z. B. 40 - 160°C), gegebenenfalls in Gegenwart eines geeigneten Katalysators, wie z. B. tert. Aminen, Metallsalzen wie Zink-2-ethylcaproat, Zinn(II)-2-ethylcaproat, Dibutylzinn(IV)-dilaurat oder Alkaliphenolat.

Geeignete Blockierungsmittel sind beispielsweise: Monophenole wie Phenol, die Kresole, die Trimethylphenole, die tert. Butylphenole; tertiäre Alkohole wie tert. Butanol, tert. Amylalkohol, Dimethylphenylcarbinol; leicht Enole bildende Verbindungen wie Acetessigester, Acetylaceton, Malonsäurederivate wie Malonsäurediester mit 1 bis 8 C-Atomen in den Alkoholresten; sekundäre aromatische Amine wie N-Methylanilin, die N-Methyltoluidine, N-Phenyltoluidin, N-Phenylxylidin; Imide wie Succinimid; Lactame wie ε -Caprolactam, δ -Valterolactam; Oxime wie Butanonoxid, Cyclohexanonoxim; Mercaptane wie Methylmercaptan, Ethylmercaptan, Butylmercaptan, 2-Mercaptobenzthiazol, α-Naphthylmercaptan, Dodecylmercaptan, Triazole wie 1H-1,2,4-Triazol.

Zur Herstellung der Lackbindemittel werden gegebenenfalls blockiertes Polyisocyanat, polyfunktioneller Reaktionspartner, Katalysator und gegebenenfalls die üblichen Zusätze, wie z. B. Pigmente, Farbstoffe, Füllstoffe und Verlaufmittel miteinander auf einem üblichen Mischaggregat, wie z. B. auf einer Sandmühle entweder mit oder ohne Lösungs- und Verdünnungsmittel gut vermischt und homogenisiert.

Die Anstrich- und Überzugsmittel können in Lösung oder aus der Schmelze, oder in fester Form nach den üblichen Methoden wie z. B. Streichen, Rollen, Gießen, Spritzen, dem Wirbelsinterverfahren oder dem elektrostatischen Pulversprühverfahren auf den zu beschichtenden Gegenstand aufgebracht werden.

Die die erfindungsgemäßen Polyisocyanate enthaltenden Lacke ergeben Filme, die überraschend gut auf metallischem Untergrund haften, besonders lichtecht, wärmefarbstabil und sehr abriebfest sind. Darüber hinaus zeichnen sie sich durch große Härte, Elastizität, sehr gute Chemikalienbeständigkeit, hohen Glanz, ausgezeichnete Wetterbeständigkeit und gute Pigmentierbarkeit aus.

Die folgenden Beispiele erläutern die Erfindung. Alle Prozentangaben betreffen Gewichtsprozente.

**Beispiele**

In den nachfolgenden Beispielen werden folgende Ausgangsmaterialien eingesetzt:

Diisocyanat 1 (1-Isocyanato-1-methyl-4-(isocyanatobut-2-yl)-cyclohexan):

a) Herstallung von 1-amino-1-methyl-4-(4-aminobut-2-yl)-cyclohexan:

830 g 3-(1-Methylcyclohexen-4-yl)-butyraldehyd werden in einem Rührautoklaven in 800 ml flüssigem Ammoniak gelöst und bei 90°C und einem Wasserstoffdruck von 100 bar über 50 g Raney-Nickel-Eisen hydriert. Nach dem Abdampfen des Lösungsmittels wird vom Katalysator abfiltriert und i. Vak. destilliert. Man erhält 720 g (86 %) 3-(1-Methylcyclohexen-4-yl)-butylamin, $Kp._{10}$ 120°C.

Bei 30 - 35°C tropft man 170 ml Blausäure hinzu, rührt 4 Stunden bei 45°C nach, destilliert i. Vak. die überschüssige Blausäure ab, fügt 300 ml Wasser hinzu, erhitzt 3 Stunden am Rückfluß, stellt mit 1400 ml 45 % Natronlauge alkalisch, dekantiert vom abgeschiedenen Salz, trennt die Phasen, extrahiert das Salz und die wäßrige Phase zweimal mit Toluol und destilliert i. Vak. Man erhalt 656 g (90 %) 1-Amino-1-methyl-4-(4-aminobut-2-yl)-cyclohexan, $Kp._{10}$ 135 - 137°C.

b) Herstellung von 1-Isocyanato-1-methyl-4-(4-isocyanatobut-2-yl)-cyclohexan:

Zu einer Lösung von 125 g Phosgen in 200 ml Dichlorbenzol tropft man bei 0°C eine Lösung von 46 g 1-Amino-1-methyl-(4-aminobut-2-yl)cyclohexan in 80 ml Dichlorbenzol. Unter Einleiten von Phosgen erwärmt man in 2 Stunden auf 150°C und leitet bei 150°C noch 3 Stunden Phosgen ein. Das Restphosgen wird mit Stickstoff ausgeblasen. Das Lösungsmittel wird im Vakuum fraktioniert. Man erhält 50 g (85 %) 1-Isocyanato-1-methyl-4-(4-isocyanatobut-2-yl)-cyclohexan, $Kp._{0,1}$ 130°C.

Diisocyanat II (1-Isocyanato-1-methyl-4-(3)-isocyanato-methylcyclohexan):

a) Herstellung von 1-Amino-1-methyl-4(3)-aminomethylcyclohexan: cyclohexan:

605 g 4(5)-Cyano-1-methylcyclohexan werden in einem Rührautoklaven in 500 ml flüssigem Ammoniak gelöst und bei 90°C und einem Wasserstoffdruck von 100 bar über 40 g Raney-Nickel-Eisen hydriert. Nach dem Abdampfen des Ammoniaks wird vom Katalysator abfiltriert und i. Vak. destilliert. Man erhält 550 g (88 %) 4(5)-Aminomethyl-1-methylcyclohexen, $Kp._{10}$ 78 - 80°C. Das Gewichtsverhältnis der 4- und 5-Isomeren liegt bei ca. 80 : 20.

Zwischen 10 und 25°C tropft man 1125 g dieses ungesättigten Amins zu 2650 g 70 % Schwefelsäure, bei 40 bis 45°C tropft man 360 ml Blausäure zu, rührt 4 Stunden bei 45°C nach, destilliert i. Vak. die nicht umgesetzte Blausäure ab, gibt 2 l Wasser hinzu, erhitzt 3 Stunden am Rückfluß, stellt mit 2,7 l 45 % Natronlauge alkalisch, dekantiert vom abgeschiedenen Salz, trennt die organische Phase ab, wäscht das Salz und die wäßrige Phase zweimal mit Toluol und destilliert über eine 80 cm-Kolonne i. Vak. Man erhält 907 g 1-Amino-1-methyl-4(3)-aminomethylcyclohexan, $Kp_{10}$ 95 - 105°C und 172 g 4(3)-Aminomethyl-1-methyl-cyclohexanol, $Kp._{10}$ 115-120°C.

b) Herstellung von 1-Isocyanato-1-methyl-4(3)-isocyanato-methylcyclohexan (1. Phosgeniermethode):

Zu einer Lösung von 250 g Phosgen in 350 ml Dichlorbenzol tropft man bei 0°C eine Lösung von 71 g 1-Amino-1-methyl-4(3)-aminomethylcyclohexan in 150 ml Dichlorbenzol. Unter Einleiten von Phosgen erwärmt man in 2 Stunden auf 150°C und leitet bei 150°C 7 Stunden Phosgen ein. Das Phosgen wird mit Stickstoff ausgeblasen, das Lösungsmittel wird i. Vak. abdestilliert und der Rückstand im Vak. fraktioniert. Man erhält 73 g 1-Isocyanato-1-methyl-4(3)-isocyanato-methylcyclohexan, $Kp._{0,1}$ 95 - 103°C. Gewichtsverhältnis der 4- bzw. 3-Isomeren ca. 80 : 20.

**Katalysator I**

2,7 g 1,4,7,10,13,16 Hexaoxacyclooctadecan und 1,0 g Kaliumacetat werden in Methanol gelöst. Nach einengen im Vakuum erhält man den Katalysator als kristallinen Komplex.

**Katalysator II**

0,5-molare Lösung von Kaliumacetat in Polyethylenglykol des mittleren Molekulargewichts 370.

**Katalysator III**

10-%-ige Lösung von 2-Hydroxypropyl-trimethylammonium-hydroxid in 2-Ethyl-1,3-dihydroxyhexan/1,3-Dihydroxybutan (Gewichtsverhältnis der Lösungsmittel: 4 : 1).

**Beispiel 1**

In eine Lösung von 250 g Diisocyanat I in 250 g wasserfreiem Toluol wird unter Feuchtigkeitsausschluß 1,0 g Katalysator I eingerührt. Das Reaktionsgemisch wird anschließend auf 80°C aufgeheizt. Nach 4-stündigem Rühren bei dieser Temperatur ist der NCO-Gehalt der Lösung von 17,8 % auf 8,8 % abgefallen. Man erhitzt kurzzeitig (15 Minuten) auf Siedetemperatur und filtriert anschließend den kristallinen Komplex ab. Die klare, gelbliche Lösung weist im Gelchromatogramm neben Toluol nur eine Hauptkomponente auf, die im Massenspektrum das Molekülion $M^{\oplus}$ = 708 (entsprechend dem Trisisocyanato-monoisocyanurat) besitzt. Der Gehalt an freiem 1-Isocyanato-4-(3-isocyanato-1-methylpropyl)-1-methylcyclohexan liegt unter 0,3 %. Nach Entfernen des Lösungsmittels erhält man ein zähviskoses Harz mit einem NCO-Gehalt von 17,6 %.

**Beispiel 2**

In eine Lösung von 250 g Diisocyanat II in 250 g wasserfreiem Toluol wird unter Feuchtigkeitsausschluß 1,0 g Katalysator I eingerührt. Anschließend wird das Reaktionsgemisch unter Rühren auf 80°C aufgeheizt. Nach 8-stündigem Rühren bei dieser Reaktionstemperatur ist der NCO-Gehalt der Lösung von 21,6 % auf 11,0 % abgefallen. Man gibt 0,39 g Benzoylchlorid hinzu, verrührt 30 Min. und filtriert dann den kristallinen Komplex ab. Die gelbliche Lösung wird vom Lösungsmittel durch Destillation befreit. Man erhält einen leicht gelblichen, festen Rückstand, der mit 100 ml n-Hexan digeriert, anschließend filtriert und mit 50 ml n-Hexan gewaschen wird.

Nach Trocknung verbleiben 224 g eines weißen Festproduktes mit einem NCO-Gehalt von 21,2 % und einem Schmelzpunkt von 128 - 130°C. Im Gelchromatogramm findet man hauptsächlich 1 Komponente, die im Massenspektrum das Molekülion $M^{\oplus}$ = 582 (entsprechend dem Tris-isocyanato-monoisocyanurat) aufweist.

**Beispiel 3**

Entsprechend Beispiel 2 werden 250 g Diisocyanat II in 250 g wasserfreiem Diisopropylether unter Verwendung von 0,5 ml des Katalysators II bei 68°C zur Umsetzung gebracht. Nach Erreichen eines NCO-Gehalts des Reaktionsansatzes von 10,4 % (Trimerisierungsgrad: 52 %) läßt man abkühlen, wobei sich ein feinkristallines, weißes Festprodukt abscheidet. Man verrührt die Suspension mit 500 ml Petrolether, filtriert und trocknet im Vakuum. Man erhält 229 g eines weißen kristallinen Festprodukts mit einem NCO-Gehalt von 20,6 % und einem Schmelzpunkt von 125 - 127°C. Aus dem Gelchromatogramm geht hervor, daß es sich

praktisch ausschließlich um das entsprechende Tris-isocyanato-monoisocyanurat handelt.

**Beispiel 4**

582 g Diisocyanat II werden bei 65°C mit 13 ml Katalysator III versetzt. Die exotherme Reaktionsmischung wird durch Kühlen bei 80°C gehalten und später auf diese Temperatur geheizt. Nach 3 h wird bei einem NCO-Gehalt von 24,7 % mit 5 ml Katalysatorlösung nachkatalysiert. Nach 6 h, als die Abnahme des NCO-Gehalts bei einem Wert von 22,1 % praktisch zum Stillstand gekommen ist (Trimerisierungsgrad: 49 %), wird die viskose Masse auf ein Blech gegossen. Man erhält ein glasartig erstarrtes Festharz mit einem NCO-Gehalt von 20,3 % und einem Schmelzbereich von 90 - 110°C. Der Gehalt an monomerem Diisocyanat liegt unter 2 %.

**Beispiel 5**

194 g Diisocyanat II werden in 48,5 g Toluol gelöst und anschließend bei 70°C mit 6 ml Katalysatorlösung versetzt. Die Abnahme des NCO-Gehalts kommt bei einer Reaktionstemperatur von 80°C nach ca. 5 h zum Stillstand (Trimerisierungsgrad: 56 %). Man erhält eine 80-%-ige Polyisocyanat-Lösung mit einem NCO-Gehalt von 14,7 % und einer Viskosität $\eta$ (23°C) = 1700 mPas bei einem Gehalt an monomerem Diisocyanat von unter 1 %.

**Patentansprüche**

1. Isocyanuratgruppen aufweisende Polyisocyanate der allgemeinen Formel

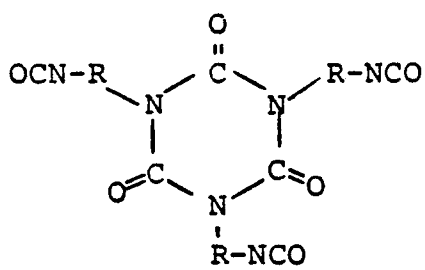

in welcher

R für einen Rest steht, wie er durch Entfernung der Isocyanatgruppen von einem aliphatisch-cycloaliphatischen Diisocyanat erhalten wird, welches eine sterisch ungehinderte, an ein primäres aliphatisches Kohlenstoffatom gebundene Isocyanatgruppe und eine sterisch gehinderte Isocyanatgruppe, die an ein tertiäres Kohlenstoffatom, das Teil eines cycloaliphatischen Ringsystems ist, aufweist, wobei die Isocyanatgruppen in der besagten Formel mit dem besagten tertiären Kohlenstoffatom verknüpft sind.

2. Isocyanato-isocyanurate der in Anspruch 1 genannten Formel, dadurch gekennzeichnet, daß R für einen Rest der Formel:

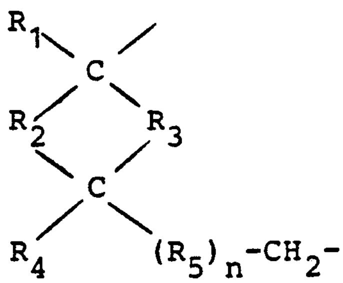

steht, wobei

$R_1$ für einen Alkylrest mit 1 bis 4 Kohlenstoffatomen,

$R_2$ und $R_3$ für gleiche oder verschiedene, lineare oder verzweigte, zweiwertige gesättigte Kohlenwasserstoffrest mit 1 bis 4 Kohlenstoffatomen,

$R_4$ für Wasserstoff oder einen Alkylrest mit 1 bis 4 Kohlenstoffatomen,

$R_5$ für einen linearen oder verzweigten, gesättigten zweiwertigen Kohlenwasserstoffrest mit 1 bis 4 Kohlenstoffatomen und

n für 0 oder 1 stehen.

3. Isocyanato-isocyanurate gemäß Anspruch 2, dadurch gekennzeichnet, daß

$R_1$ für einen Methylrest steht,

$R_2$ und $R_3$ gleiche oder verschiedene lineare, zweiwertige, gesättigte Kohlenwasserstoffreste mit 1 bis 3 Kohlenstoffatomen bedeuten, mit der Maßgabe daß die Summe der Anzahl der Kohlenstoffatome der Reste $R_2$ und $R_3$ 3 oder 4 beträgt,

$R_4$ für ein Wasserstoffatom steht und

$R_5$ und n die in Anspruch 2 genannte Bedeutung haben.

4. Verfahren zur Herstellung von Isocyanuratgruppen aufweisenden Polyisocyanaten durch katalytische Trimerisierung eines Teils der Isocyanatgruppen von organischen Diisocyanaten und gegebenenfalls Abstoppung der Trimerisierungsreaktion beim jeweils gewünschten Trimerisierungsgrad durch Zugabe eines Katalysatorengifts und/oder durch thermische Desaktivierung des eingesetzten Katalysators, dadurch gekennzeichnet, daß man als Ausgangsdiisocyanate solche einsetzt, die bei einem NCO-Gehalt von 20 bis 50 Gew.-% eine sterisch ungehinderte, an ein primäres aliphatisches Kohlenstoffatom gebundene Isocyanatgruppe und eine sterisch gehinderte Isocyanatgruppe, die an ein tertiäres Kohlenstoffatom, das Teil eines cycloaliphatischen Ringsystems ist, aufweisen.

5. Verfahren gemäß Anspruch 4, dadurch gekennzeichnet, daß man als Ausgangsdiisocyanate solche der allgemeinen Formel:

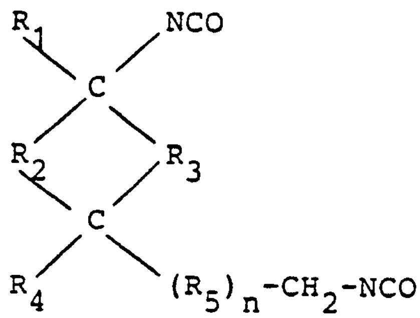

einsetzt, wobei

$R_1$, $R_2$, $R_3$, $R_4$, $R_5$ und n die in Anspruch 2 genannte Bedeutung haben.

6. Verfahren gemäß Anspruch 4 und 5, dadurch gekennzeichnet, daß man die Trimerisierungsreaktion bei Erreichen eines Trimerisierungsgrads von 50 % abbricht.

7. Verwendung der Isocyanuratgruppen aufweisenden Polyisocyanate gemäß Ansprüchen 1 bis 3, gegebenenfalls in mit Blockierungsmitteln für Isocyanatgruppen blockierter Form, als Isocyanatkomponente in Polyurethanlacken.

**Claims**

1. Polyisocyanates containing isocyanurate groups corresponding to the following formula:

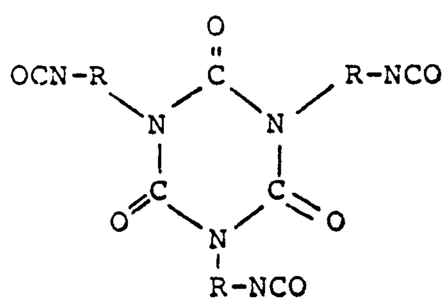

wherein

R stands for a group obtained by removal of the isocyanate groups from an aliphatic-cycloaliphatic diisocyanate which contains a sterically unhindered isocyanate group attached to a primary aliphatic carbon atom and a sterically hindered isocyanate group attached to a tertiary carbon atom which forms part of a cycloaliphatic ring system, the isocyanate groups in the said formula being attached to the said tertiary carbon atom.

2. Isocyanato-isocyanurates corresponding to the formula indicated in claim 1, characterised in that R stands for a residue of the formula:

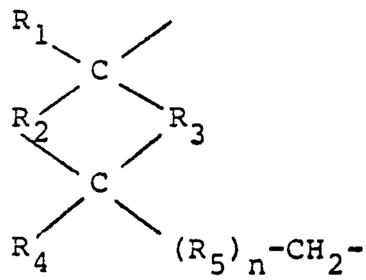

wherein

$R_1$ denotes an alkyl group with 1 to 4 carbon atoms,

$R_2$ and $R_3$ denote identical or different, straight chained or branched, divalent saturated hydrocarbon groups with 1 to 4 carbon atoms,

$R_3$ denotes hydrogen or an alkyl group with 1 to 4 carbon atoms,

$R_5$ denotes a straight chained or branched, saturated divalent hydrocarbon group with 1 to 4 carbon atoms and n stands for 0 or 1.

3. Isocyanato-isocyanurates according to claim 2, characterised in that

$R_1$ stands for a methyl group,

$R_2$ and $R_3$ denote identical or different straight chained, divalent, saturated hydrocarbon groups with 1 to 3 carbon atoms under the condition that the sum of the number of carbon atoms of groups $R_2$ and $R_3$ is 3 or 4, R stands for a hydrogen atom and

$R_4$ and n have the meanings indicated in claim 2.

4. Process for the preparation of polyisocyanates containing isocyanurate groups by the catalytic trimerisation of a proportion of the isocyanate groups of organic diisocyanates and optionally stopping of the trimerisation reaction at the desired degree of trimerisation by the addition of a catalystpoison and/or by thermal inactivation of the catalyst put into the process, characterised in that the diisocyanates used as starting material have an NCO content of from 20 to 50 % by weight and contain a sterically unhindered isocyanate group attached to a primary aliphatic carbon atom and a sterically hindered isocyanate group on a tertiary carbon atom which forms part of a cycloaliphatic ring system.

5. Process according to claim 4, characterised in that the diisocyanates used as starting material correspond to the following general formula:

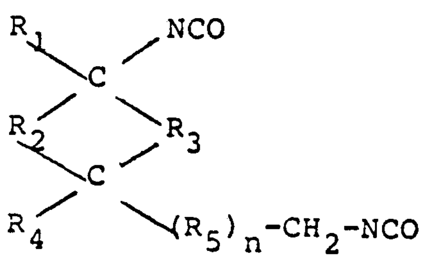

wherein

$R_1$, $R_2$, $R_3$, $R_4$, $R_5$ and n have the meanings indicated in claim 2.

6. Process according to claims 4 and 5, characterised in that the trimerisation reaction is stopped when a degree of trimerisation of 50 % has been reached.

7. Use of the polyisocyanates containing isocyanurate groups according to claims 1 to 3, optionally blocked with blockingagents for isocyanate groups, as isocyanate component in polyurethane lacquers.

**Revendications**

1. Polyisocyanates porteurs de groupes isocyanurate, de formule générale:

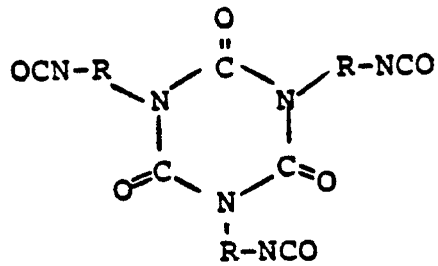

dans laquelle

R représente un reste tel qu'on en obtient en enlevant les groupes isocyanato d'un diisocyanate aliphato-cycloaliphatique qui porte un groupe ésocyanato sous enconu brement stérique, lié à un atome aliphatique primaire de carbone et un groupe isocyanato à encombrement stérique lié à un atome tertiaire de carbone qui fait partie d'un noyau cycloaliphatique, les groupes isocyanato dans ladite formule étant en liaison avec ledit atome tertiaire de carbone.

2. Isocyanato-isocyanurates de formule suivant la revendication 1, caractérisés en ce que R représente un reste de formule:

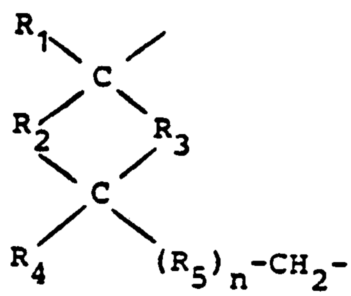

dans laquelle

$R_1$ est un reste alkyle ayant 1 à 4 atomes de carbone,

$R_2$ et $R_3$ sont des restes hydrocarbonés saturés divalents linéaires ou ramifiés, identiques ou différents, ayant 1 à 4 atomes de carbone,

$R_4$ est l'hydrogèns ou un reste alkyle ayant 1 à 4 atomes de carbone,

$R_5$ est un reste hydrocarboné divalent saturé linéaire ou ramifié ayant 1 à 4 atomes de carbone et

n a la valeur 0 ou 1.

3. Isocyanato-isocyanurates suivant la revendication 2, caractérisés en ce que

$R_1$ représente un reste méthyle,

$R_2$ et $R_3$ dont des restes hydrocarbonés linéaires saturés divalents, identiques ou différents, ayant 1 à 3 atomes de carbone, sous réserve que la somme des nombres d'atomes de carbone des restes $R_2$ et $R_3$ ait la valeur 3 ou 4,

$R_4$ est un atome d'hydrogène et

$R_5$ et n ont la définition mentionnée dans la revendication 2.

4. Procédé de production de polyisocyanates porteurs de groupes isocyanurate par trimérisation catalytique d'une partie des groupes isocyanato de diidocyanates organiques et, le cas échéant, arrêt de la réaction de trimérisation pour tout degré désiré de trimérisation par addition d'un poison du catalyseur et/ou par désactivation thermique du catalyseur utilisé, caractérisé en ce qu'on utilise comme diisocyanates de départ des diisocyanates qui portent, pour une teneur en NCO de 20 à 50 % en poids, un groupe isocyanato sans encombrement stérique, lié à un atome aliphatique primaire de carbone et un groupe isocyanato à encombrement stérique lié à un atome tertiaire de carbone qui fait partie d'un noyau cycloaliphatique.

5. Procédé suivant la revendication 4, caractérisé en ce qu'on utilise comme diisocyanates de départ des diisocyanates de formule générale:

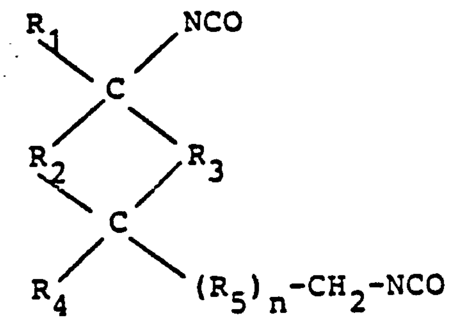

dans laquelle

$R_1$, $R_2$, $R_3$, $R_4$, $R_5$ et n ont la définition mentionnée dans la revendication 2.

6. Procédé suivant les revendications 4 et 5, caractérisé en ce qu'on arrête la réaction de trimérisation lorsqu'un degré de trimérisation de 50 % a été atteint.

7. Utilisation des polyisocyanates porteurs de groupes isocyanurate suivant les revendications 1 à 3, le cas échéant sous une forme bloquée, avec des agents de protection de groupes isocyanato, comme composant isocyanate dans des laques à base de polyuréthanne.